# EUROPÄISCHE PATENTSCHRIFT

(11) **EP 2 272 505 B1**
(45) Veröffentlichungstag und Bekanntmachung des Hinweises auf die Patenterteilung: **12.10.2016**
(21) Anmeldenummer: 10183693.0
(22) Anmeldetag: 03.07.2003
(51) Int. Cl.: A61K 9/28, A61K 31/53, A61P 15/10

(54) **Vardenafil Hydrochlorid Trihydrat enthaltende Tabletten**
Tablets containing vardenafil hydrochloride trihydrate
Comprimés contenant du hydrochlorure de vardénafil trihydrate

(30) Priorität: 16.07.2002 DE 10232113
(43) Veröffentlichungstag der Anmeldung: 12.01.2011
(62) Teilanmeldung aus: 03763695.8
(73) Patentinhaber: Bayer Intellectual Property GmbH, 40789 Monheim (DE)
(72) Erfinder: Serno, Peter, 51467 Bergisch Gladbach (DE); Grunenberg, Alfons, 41539 Dormagen (DE); Ohm, Andreas, 41468 Neuss (DE); Bellinghausen, Rainer, 51519 Odenthal (DE); Volters, Elmer, 51373 Leverkusen (DE); Henck, Jan-Olav, 47 906 West Lafayett, IN (US)
(74) Vertreter: BIP Patents

(56) Entgegenhaltungen:
- WO-A-01/19357
- GB-A- 790 762
- US-B1- 6 362 178

## Beschreibung

Die Anmeldung betrifft überzogene Tabletten, welche Vardenafil Hydrochlorid zu mindestens 90 Mol-% als Trihydrat in fester Form enthalten, und Verfahren zu ihrer Herstellung.

Der pharmazeutische Wirkstoff Vardenafil (IUPAC-Name: 2-Ethoxy-5-[(4-ethyl-1-piperazinyl)sulfonyl]phenyl}-5-methyl-7-propylimidazo[5,1-*f*][1,2,4]triazin-4(3*H*)-on), Vardenafil Hydrochlorid und Vardenafil Hydrochlorid Trihydrat und deren Verwendung zur Behandlung der Erektilen Dysfunktion werden in der WO 99/24433 als Beispiele 19, 20 bzw. 336 beschrieben.

Es wurde gefunden, dass Vardenafil Hydrochlorid in vier verschiedenen polymorphen Formen auftritt (wasserfreie Modifikationen I mit Schmelzpunkt 217°C, Modifikation II mit Schmelzpunkt 190°C, Modifikation III mit Schmelzpunkt 183-186°C, Modifikation IV mit Umwandlungspunkt 166°C) und dass sich bei Raumtemperatur keine dieser polymorphen Formen bevorzugt bildet. Des Weiteren können die einzelnen polymorphen Formen in Abhängigkeit von der Umgebungsfeuchte und Temperatur unterschiedliche Wassermengen aufnehmen und mit Wasser weitere polymorphe Formen, sogenannte pseudopolymorphe Formen bilden.

Da verschiedene polymorphe Formen einer Substanz sich häufig hinsichtlich ihres Lösungsverhaltens unterscheiden, können sich diese Unterschiede z.B. in der Bioverfügbarkeit (AUC), maximalen Plasmakonzentration (Cₘₐₓ) und dem Zeitpunkt des Auftretens der maximalen Plasmakonzentration (AUCmax) äußern. Auch eine Minderresorption mit der Folge einer unzureichenden oder gänzlich ausbleibenden Wirkung ist möglich.

Es besteht daher einerseits das Problem, dass feste Arzneimittel, insbesondere überzogene Tabletten den Wirkstoff Vardenafil Hydrochlorid in definierter und reproduzierbarer Form enthalten müssen. Andererseits sind aber die polymorphen Formen von Vardenafil HCl nicht in reiner Form herstellbar bzw. isolierbar, da sie jeweils geringe Mengen Wasser aufnehmen und somit als Mischung der polymorphen Form und eines Hydrates vorliegen.

Aus diesen Gründen eignet sich Vardenafil Hydrochlorid nicht als Inhaltsstoff von Arzneimitteln, insbesondere überzogenen Tabletten, in denen der Wirkstoff in fester Form vorliegen soll.

Es wurde nun gefunden, dass bei Verwendung von Vardenafil Hydrochlorid in Form des Trihydrats feste Arzneimittel, insbesondere überzogene Tabletten in einheitlicher und reproduzierbarer Form erhalten werden können, wenn diese überzogenen Tabletten nach ihrer Herstellung befeuchtet werden.

Gegenstand der Erfindung ist daher ein Verfahren zur Herstellung von überzogenen Tabletten enthaltend Vardenafil Hydrochlorid Trihydratmit einem Trihydrat-Gehalt von mindestens 90 Mol-%, dadurch gekennzeichnet, dass
a) bei der Herstellung der überzogenen TablettenVardenafil Hydrochlorid mit beliebigem Kristallwassergehalt eingesetzt wird, und
b) im Endprodukt das Vardenafil Hydrochlorid in die Trihydratform überführt wird indem das Endprodukt so lange mit befeuchtetem Gas in Kontakt gebracht werden, bis sich zu mindestens 90 Mol-% das Trihydrat gebildet hat.

In diesem Verfahren kann Vardenafil Hydrochlorid mit einem beliebigen Wassergehalt, d.h. als Trihydrat oder in einer Form, deren Kristallwassergehalt wesentlich von 9,3 Gewichts-% abweicht, eingesetzt werden.

Im Endprodukt wird Vardenafil Hydrochlorid in die Trihydratform überführt.

Die Überführung in die Trihydratform erfolgt erfindungsgemäß dadurch, dass das Endprodukt in einer geeigneten Anlage so lange mit befeuchtetem Gas in Kontakt gebracht werden, bis sich zu mindestens 90 Mol-% das Trihydrat gebildet hat Befeuchtetes Gas ist insbesondere Luft mit einer relativen Feuchte von 35 % bis 100 %, besonders bevorzugt 50 % bis 99 %. Geeignete Anlagen sind alle Anlagen oder Räume in denen das befeuchtete Gas eingeleitet oder bereit gestellt werden kann und mit den Arzneiformen möglichst gleichmäßig in Kontakt gebracht werden kann oder in denen die Arzneiformen bei den genannten Bedingungen inkubiert werden können. Die Über- oder Einleitdauer mit dem befeuchteten Gas beziehungsweise die Verweildauer der Arzneiform in der Anlage und die relative Feuchte des Gases hängt vom Ausgangswassergehalt der Arzneiform und dem Mengenverhältnis zwischen befeuchtetem Gas und Arzneiform ab. Sie kann von wenigen Minuten bis zu mehreren Tagen variieren, in den meisten Fällen sind 0,5 - 12 Stunden ausreichend. Bei verpackten Arzneiformen sind im Allgemeinen Zeiten von 1 Tag - 6 Monaten ausreichend.

Feste Arzneimittel im Sinne der Erfindung sind überzogene Tabletten, da bei der Verarbeitung von Vardenafil Hydrochlorid Trihydrat zur Herstellung von Tabletten, insbesondere beim Überziehen der Tabletten, nach den üblichen Methoden in besonderem Maße das Trihydrat wieder teilweise oder vollständig dehydratisiert wird und der Wirkstoff wieder uneinheitlich in mehreren polymorphen und pseudopolymorphen Formen vorliegt.

Neben Vardenafil Hydrochlorid Trihydrat, das bei Raumtemperatur in nur einer Kristallmodifikation vorliegt (Wassergehalt im Vardenafil Hydrochlorid Trihydrat beträgt 9,3 Gewichts-%) enthalten die erfindungsgemäß hergestellten überzogenen Tabletten weitere dem Fachmann bekannte pharmazeutische Hilfsstoffe.

Die erfindungsgemäßen überzogenen Tabletten enthalten zusätzlich zum Vardenafil Hydrochlorid Trihydrat vorzugsweise Füllmittel, Sprengmittel und Schmiermittel und gegebenenfalls weitere Hilfsstoffe. Die erfindungsgemäßen überzogenen Tabletten enthalten vorzugsweise Vardenafil Hydrochlorid Trihydrat zu 0,1 - 70 Gewichts-%, das Sprengmittel zu 0,1 - 10 Gewichts-%, das Schmiermittel zu 0,1 - 2 Gewichts-% und gegebenenfalls weitere Hilfsmittel sowie das Füllmittel als restliche Bestandteile.

Als Füllmittel enthält die überzogene Tablette vorzugsweise mikrokristalline Cellulose, als Sprengmittel vorzugsweise Crospovidon, als Schmiermittel vorzugsweise Magnesiumstearat.

Weitere Hilfsmittel, die gegebenenfalls der überzogenen Tablette zugefügt werden können, sind beispielhaft und vorzugsweise Fließreguliermittel wie hochdisperses Siliciumdioxid.

Besonderes bevorzugt sind überzogene Tabletten, die auch in applikationsfertiger Form den Wirkstoff Vardenafil Hydrochlorid in reproduzierbarer Weise in der Modifikation des Trihydrates enthalten.

Überzogene Tabletten sind durch das Europäische Arzneibuch, 3. Ausgabe 1997, Seite 1852, eindeutig definiert: "Überzogene Tabletten sind Tabletten, die mit einer Schicht oder mehreren Schichten von Mischungen verschiedener Substanzen, z.B. mit natürlichen oder synthetischen Harzen, Gummen, Gelatine, inaktiven und unlöslichen Füllmitteln, Zuckern, Weichmachern, Polyolen, Wachsen, zugelassenen Farbmitteln sowie gegebenenfalls Geschmackskorrigentien und Wirkstoffen überzogen sind. Die Substanzen, die als Überzug dienen, werden normalerweise in Lösung oder als Suspension unter Bedingungen, bei denen das Lösungs- oder Dispersionsmittel verdunstet, aufgebracht. Ist der Überzug ein sehr dünner Polymerüberzug, werden die Tabletten als Filmtabletten bezeichnet."

Ferner wird auf die Definition von Tabletten im Europäischen Arzneibuch, 3. Ausgabe 1997 Bezug genommen.

Da während des Prozesses das Lösungs- oder Dispergiermittel abzutrocknen und der Lack zu verfilmen ist, tritt bei der Herstellung von überzogenen Tabletten enthaltend Vardenafil Hydrochlorid Trihydrat nach den üblichen Verfahren ein Verlust an Kristallwasser des Wirkstoffes in besonders hohem Maße ein.

Eine bevorzugte Ausführungsform der Erfindung ist daher ein Verfahren in dem überzogene Vardenafil Hydrochlorid Trihydrat Tabletten hergestellt werden, welche auch in applikationsfertiger Form Vardenafil Hydrochlorid in reproduzierbarer Weise in der Form des Trihydrates enthalten.

In diesem bevorzugten Verfahren werden die überzogenen Vardenafil HCl Tabletten einem Rehydratisierverfahren unterworfen. Dabei bildet sich in allen Fällen in der überzogenen Tablette überraschenderweise ein und dasselbe Trihydrat von Vardenafil HCl und zwar unabhängig davon, welche polymorphe Form oder Mischung polymorpher Formen von Vardenafil Hydrochlorid zunächst in der überzogenen Tablette enthalten war.

Ein weiterer Gegenstand der Erfindung ist daher ein Verfahren zur Herstellung von überzogenen Tabletten enthaltend Vardenafil Hydrochlorid Trihydrat, das dadurch gekennzeichnet ist, dass nach üblichen Methoden hergestellte überzogene Tabletten enthaltend Vardenafil Hydrochlorid in einer oder mehreren beliebigen (Hydrat) Modifikationen, einem Rehydratisierungsprozess unterworfen werden.

Der Rehydratisierungsprozess wird vorzugsweise so durchgeführt, dass die überzogenen Tabletten in einer geeigneten Anlage so lange mit befeuchtetem Gas in Kontakt gebracht werden bis sich in der Arzneiform das Trihydrat von Vardenafil HCl zu mindestens 90 Mol-% gebildet hat.

Geeignete Anlagen in denen der Rehydratisierungsprozess durchgeführt wird, sind beispielhaft und vorzugsweise Klimaschränke, Klimaräume, Wirbelschichtgranulatoren, Lackiergeräte oder Trommeln.

Befeuchtetes Gas ist beispielhaft und vorzugsweise Luft mit einer relativen Feuchte von 35 % bis 100 %, besonders bevorzugt 50 % bis 99 %.

Während des Rehydratisiervorganges können die überzogenen Tabletten in der Anlage ruhen, beispielsweise auf Hordenblechen eines Klimaschrankes oder auf dem Bodenblech eines Wirbelschichtgranulators oder auch zur besseren Durchmischung ständig oder gelegentlich bewegt werden, beispielsweise in der Trommel einer Lackieranlage. Es ist auch möglich, den Rehydratisierprozess nach Verpackung der überzogenen Tabletten in einer wasserdampfdurchlässigen Verpackung durchzuführen. Hierzu werden die verpackten überzogenen Tabletten in einem Klimaraum inkubiert.

Die Zeitdauer der Rehydratisierung hängt vom Ausgangswassergehalt der überzogenen Tablette, der relativen Feuchte des befeuchteten Gases und dem Mengenverhältnis zwischen befeuchtetem Gas und der überzogenen Tablette ab. Sie kann von wenigen Minuten bis zu mehreren Tagen variieren, in den meisten Fällen sind 0,5 - 12 Stunden Rehydratisierzeit ausreichend. Im Fall der Rehydratisierung in wasserdampfdurchlässiger Verpackung hängt die Rehydratisierdauer zusätzlich von der Wasserdampfdurchlässigkeit des Packmittels ab. In pharmazeutisch üblichen Blisterpackungen sind im Allgemeinen Zeiten von 1 Tag bis zu 6 Monaten ausreichend.

Erstaunlicherweise ist es durch das erfindungsgemäße Verfahren möglich, auch noch im dichten Gefüge der fertigen lackierten Tablette undefinierte Modifikationsgemische von Vardenafil Hydrochlorid vollständig in die Trihydratform zu überführen. Dabei treten keine unerwünschte Nebeneffekte auf die Qualität der überzogenen Tablette ein, wie beispielsweise ein Zerbröseln, ein Aufplatzen von Lackschichten oder eine Verminderung der Freisetzungsgeschwindigkeit des Wirkstoffes.

Es wurde auch gefunden, dass die nach dem erfindungsgemäßen Verfahren hergestellten Tabletten gegenüber überzogenen Tabletten, die unter Verwendung von Vardenafil Hydrochlorid Trihydrat in üblichen Verfahren hergestellt werden, eine Reihe von Vorteilen haben.

Gegenstand der Anmeldung sind daher auch die nach dem erfindungsgemäßen Verfahren hergestellten überzogenen Tabletten.

Der Vorteil der erfindungsgemäßen bzw. nach dem erfindungsgemäßen Verfahren hergestellten überzogenen Tabletten ist, dass die Kristallstruktur des Wirkstoffes im Arzneimittel eindeutig definiert, reproduzierbar und über einen weiten Bereich relativer Luftfeuchten stabil ist, entsprechende überzogene Tabletten weisen einen rascheren Zerfall auf und entgegen der Erwartung aus dem bisherigen Stand der Technik (David J.W. Grant, T. Higuchi, Techniques of Chemistry, Volume XXI, Seiten 38, 42 und 43) ist die Auflösegeschwindigkeit des Wirkstoffes aus den erfindungsgemäßen bzw. nach dem erfindungsgemäßen Verfahren hergestellten überzogenen Tabletten unverändert schnell.

Die erfindungsgemäßen bzw. nach dem erfindungsgemäßen Verfahren hergestellten überzogenen Tabletten können anhand der Kristallstruktur durch das Raman-Spektrum (das FT-Ramanspektrum von Vardenafil Hydrochlorid Trihydrat zeigt einen signifikanten Peak bei 1701 cm⁻¹. Wasserärmere polymorphe und pseudo-polymorphe Formen und deren Mischungen haben dagegen eine Bande bei 1692 cm⁻¹), IR - Spektrum, NIR - Spektrum, FIR - Spektrum, ¹³C Festkörper NMR-Spektrum und das Röntgendiffraktogramm eindeutig definiert werden (vgl. Abbildungen 2, 4 - 8 und Tab. 3 - 8 in der Anlage).

Bezüglich der Herstellung von Vardenafil Hydrochlorid und Vardenafil Hydrochlorid Trihydrat wird auf die Offenbarung der WO 99/24433, insbesondere auf die Beispiele 20 bzw. 336, hiermit ausdrücklich Bezug genommen.

Erfindungsgemäß besteht das Vardenafil Hydrochlorid in den überzogenen Tabletten zu mindestens 90 Mol-%, besonders bevorzugt zu mindestens 95 Mol-% aus der Trihydratform.

Die erfindungsgemäßen bzw. nach dem erfindungsgemäßen Verfahren hergestellten überzogenen Tabletten eignen sich zur Behandlung von Krankheiten bei Menschen und/oder Tieren, insbesondere zur Behandlung sexueller Dysfunktionen, ganz besonders zur Behandlung der erektilen Dysfunktion.

Die erfindungsgemäßen überzogenen Tabletten weisen mehrere, unerwartete Vorteile auf:
1. Die überzogenen Tabletten enthalten Vardenafil Hydrochlorid nur in einer Kristallmodifikation. Die überzogenen Tabletten lassen sich daher reproduzierbar herstellen, und sie setzen Vardenafil mit einer reproduzierbaren und einheitlichen Geschwindigkeit frei.
2. Die Freisetzungsgeschwindigkeit des Wirkstoffs aus den erfindungsgemäßen überzogenen Tabletten ist vergleichbar mit der aus überzogenen Tabletten, die den Wirkstoff solvatfrei enthalten. Gewöhnlich ist die Freisetzungsgeschwindigkeit aus solvathaltigen, hier wasserhaltigen, Kristallen im selben Solvens, hier Wasser, kleiner als aus solvatfreien Kristallen.
3. Die Zerfallszeit der überzogenen Tabletten, ist kürzer. Die überzogenen Tabletten sind daher besonders für die Behandlung von Krankheiten geeignet, bei denen der schnelle Wirkeintritt erwünscht ist, wie beispielsweise bei der Behandlung der erektilen Dysfunktion.
4. Im Gegensatz zu den stark hygroskopischen Arzneimitteln, welche solvatfreies Vardenafil Hydrochlorid enthalten, sind die erfindungsgemäßen überzogenen Tabletten über lange Zeiten lagerstabil und verändern ihre Zusammensetzung, insbesondere ihren Wassergehalt, kaum.

Die Applikation der erfindungsgemäßen überzogenen Tabletten kann auf unterschiedliche Weise erfolgen. Beispielsweise seien genannt: oral, sublingual, bukkal, nasal, inhalativ, subcutan, oder topisch. Bevorzugt ist die orale Applikation.

Im Allgemeinen hat es sich als vorteilhaft erwiesen, Mengen von etwa 0,001 bis 10 mg/kg, bei oraler Anwendung vorzugsweise etwa 0,005 bis 3 mg/kg Körpergewicht zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art dessen Formulierung und dem Zeitpunkt bzw. Intervall, zu welchen die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Beispiele dienen zur weiteren Erläuterung der Erfindung, die Erfindung ist nicht auf die Beispiele beschränkt.

### Beispiele

### Vergleichsbeispiel 1 und Beispiel 2: Tabletten aus üblichem Herstellungsverfahren der Walzgranulation und Lackierung und verbesserter Zerfall erfindungsgemäßer Tabletten

216 g mikrofeines Vardenafil HCl werden mit 605 g mikrokristalliner Cellulose und 43,2 g Crospovidone gemischt. Nach Zusatz von 2101 g mikrokristalliner Cellulose und 132 g Crospovidone wird gemischt und mit 350 g mikrokristalliner Cellulose, 17,5 g hochdispersem Siliciumdioxid und 35 g Magnesiumstearat nachgemischt. Die Mischung wird zu Tabletten von 6 mm Durchmesser und einer Masse von 87 mg (entspr. 5 mg Vardenafil Base) auf einer Rundläuferpresse verpresst. In einem Lackiergerät werden pro Tablette 43,5 mg einer Lacksuspension aus 4,5 % Hypromellose, 1,5 % Macrogol 400, 1,23 % Titandioxid, 0,25 % Eisenoxid gelb und 0,02 % Eisenoxid rot aufgesprüht.

### Vergleichsbeispiel 1

Vardenafil Hydrochlorid liegt in den Tabletten anteilig als Trihydrat und Anhydrat (1 bis 4 Modifikationen) vor. Die Tabletten haben eine Zerfallszeit von 2 Minuten.

### Beispiel 2

Die Tabletten werden in einem Wirbelschichtgranulator über 4 Stunden bei 150 m³/h Zuluft von 30°C und 19 g/kg Wassergehalt rehydratisiert (entspricht 70 % relativer Luftfeuchte). Erfindungsgemäß ***entspricht*** dadurch die Modifikation des Vardenafil HCl in der Tablette dem Trihydrat. Die Tabletten weisen nur noch eine Zerfallszeit von ½ Minute auf.

### Beispiel 3: Stabilität erfindungsgemäß hergestellter Tabletten

28,4 kg mikronisiertes Vardenafil HCl Trihydrat wird mit 69,6 kg mikrokristalliner Cellulose und 5,16 kg gesiebtem Crospovidone in einem Zwangsmischer gemischt. Die Mischung wird mit 182 kg mikrokristalliner Cellulose und 9,84 kg Crospovidone in einem Containermischer gemischt, gesiebt und durch trockene Walzgranulation granuliert. Nach Nachmischen mit 1,50 kg hoch dispersem Siliciumdioxid und 3,00 kg Magnesiumstearat wird auf einer Rundlauftablettenpresse zu Tabletten mit einer Masse von 125 mg und einem Durchmesser von 7 mm tablettiert. Die unlackierten Tabletten werden in einer handelsüblichen Lackieranlage mit einer Suspension aus 5,74 kg Hypromellose, 1,91 kg Macrogol 400, 1,57 kg Titandioxid, 319 g gelbem Eisenoxid, 25,5 g rotem Eisenoxid und 118 kg gereinigtem Wasser lackiert. Die Modifikation des Vardenafil HCl in den lackierten Tabletten ***entspricht nicht*** der Trihydratform und stellt daher ein undefiniertes Gemisch wasserfreier und wasserhaltiger Formen von Vardenafil HCl dar. Die lackierten Tabletten werden in der Lackieranlage 5 Stunden lang bei 25°C mit Luft behandelt, die einen Wassergehalt von 16 g Wasser / kg aufweist (entspricht 80 % relativer Luftfeuchte). Die Modifikation des Vardenafil HCl in den rehydratisierten Tabletten ***entspricht*** dem Trihydrat.

Stabilitätstest: Die Tabletten mit Vardenafil HCl Trihydrat werden eine Woche bei 25°C und 30 % relativer Feuchte offen gelagert. Trotz der geringen Luftfeuchte tritt dabei kein Kristallwasserverlust ein und die Wirkstoffmodifikation des Vardenafil HCl ***entspricht*** nach wie vor der Trihydratform.

### Beispiel 4: Wirkstofffreisetzung aus erfindungsgemäß hergestellten Tabletten

336 g Vardenafil HCl wird mit 2216 g mikrokristalliner Cellulose und 134 g Crospovidone gemischt und trocken granuliert. Nach Versetzen des Granulates wird mit 283 g mikrokristalliner Cellulose, 16 g Crospovidone und 15 g Magnesiumstearat nachgemischt und zu Tabletten von 5 mm Durchmesser und einer Masse von 48 mg verpresst (entsprechend 5 mg Vardenafil Base pro Tablette). Die Tabletten werden mit 57,4 g Hypromellose, 19,1 g Macrogol 4000 und 19,1 g Titandioxid weiß lackiert und 4 Tage lang bei 25°C und 80 % relativer Luftfeuchte offen gelagert. Die Modifikation des Vardenafil HCl in der Tablette ***entspricht*** der Trihydratform.

Wie die Freisetzungsdaten der Tabelle 1 zeigen, weisen die so hergestellten erfindungsgemäßen Tabletten trotz der vollständigen Wiederherstellung der Trihydratform von Vardenafil HCl in der fertigen Tablette eine extrem rasche Wirkstofffreisetzung auf.

**Tabelle 1: Wirkstofffreisetzung aus Tabletten des Beispiel 4**

| Zeit | Wirkstofffreisetzung |
|---|---|
| 5 min | 100 % |
| 10 min | 101 % |
| 30 min | 101 % |
| 45 min | 101 % |

| | |
|---|---|
| Freisetzungsbedingungen: n = 6, USP Paddle, 900 ml 0.1 - M - HCl, 75 rpm, 10 µm Filter | |

### Beispiel 5 Geringe Streuung der relativen Bioverfügbarkeit erfindungsgemäßer Tabletten

0,645 kg Vardenafil HCl werden mit 2,42 kg mikrokristalliner Cellulose und 161 Crospovidone gemischt, gesiebt und mit einer Walze trocken granuliert. Das Granulat wird mit 0,339 kg mikrokristalliner Cellulose, 18,8 g Crospovidone und 18 g Magnesiumstearat nachgemischt und zu runden Tabletten von 7 mm Durchmesser und einer Masse von 120 mg (entsprechend 20 mg Vardenafil Base) verpresst. Die Tabletten werden mit 0,765 mg Macrogol 4000, 2,295 mg Hypromellose und 0,765 mg Titandioxid (Mengen jeweils pro Tablette) lackiert. Zur Herstellung der Trihydrat-form von Vardenafil HCl in den fertigen Tabletten werden diese 72 Stunden lang auf Blechen in einem Klimaraum von 16 - 24°C und 60 - 75 % relativer Feuchte ausgelegt.

Zu Vergleichszwecken wird eine Lösung bestehend aus 21,49 mg Vardenafil HCl (entspr. 20 mg Vardenafil Base), 38,69 mg Methylparahydroxybenzoat, 4,298 mg Propylparahydroxybenzoat, 6448 mg Saccharose, 17419 mg Wasser und Milchsäure ad pH 3,9 hergestellt. Die Pharmakokinetik nach Gabe von Tabletten und Lösung wurden in einer randomsierten, offenen Crossover - Studie an 12 männlichen Probanden verglichen (Tabelle 2).

**Tabelle 2: Pharmakokinetische Parameter nach Gabe von Tabletten des Beispiel 5 und einer Lösung (geometrische Mittel / geometrische Standardabweichungen)**

| Parameter | Tabletten entspr. Beispiel 5 | Vergleichslösung |
|---|---|---|
| AUC ( µg x h/L) | 60,2 / 1,64 | 64,6 / 1,78 |
| Cmax (µg / L) | 21,1 / 1,86 | 22,5 / 2,09 |

Die Ergebnisse zeigen, dass erfindungsgemäße Tabletten eine relative Bioverfügbarkeit von 93 % im Vergleich zu einer wässrigen Lösung aufweisen. Da die geometrischen Standardabweichungen der Bioverfügbarkeit und maximalen Plasma-Konzentration kleiner sind als nach Gabe der wässrigen Lösung, können Streuungen infolge variabler polymorpher oder pseudopolymorpher Formen von Vardenafil HCl in Feststoffform in der Tablette ausgeschlossen werden.

### Beispiel 6

0,871 kg Vardenafil HCl Trihydrat, 2,13 kg mikrokristalline Cellulose und 0,158 kg gesiebtes Crospovidone werden in einem Pflugscharmischer intensiv gemischt. Diese Mischung wird mit 3,08 kg mikrokristalliner Cellulose und 0,167 kg Crospovidone gemischt, walzgranuliert und mit 0,0325 kg hochdispersem Siliciumdioxid und 0,0650 kg Magnesiumstearat nachgemischt.

Die Mischung wird auf einer Rundlaufpresse zu Tabletten von 8 mm Durchmesser und einer Masse von 177 mg (entsprechend 20 mg Vardenafil Base) tablettiert und mit 2,76 kg einer wässrigen Lacksuspension mit 4,5 % Hypromellose, 1,5 % Macrogol 400, 1,23 % Titandioxid, 0,25 % Eisenoxid gelb und 0,02 % Eisenoxid rot in einer Lackiertrommel lackiert. Die Modifikation des Vardenafil HCl in den Tabletten ***entspricht nicht*** der Trihydratform (Abbildung 1a).

Die Rehydratisierung wird durchgeführt indem die lackierten Tabletten in einen Wirbelschichtgranulator vom Typ Glatt GPCG 1/3 eingebracht werden und mit 150 m³/h Zuluft von 25°C und 16 g/kg Feuchte (entsprechend 80 % relativer Luftfeuchte) für 4 Stunden behandelt werden. Die Modifikation des Vardenafil HCl in den so behandelten Tabletten ***entspricht*** der Trihydratform (Abbildung 1b).
**Abbildung 1a** Differenz-Ramanspektrum (= Spektrum Tablette - Spektrum Vardenafil HCl 3 H2O) von Tabletten des Beispieles 6 vor Rehydratisierung Vardenafil HCl wasserfrei nicht rehydratisierte Tabletten. 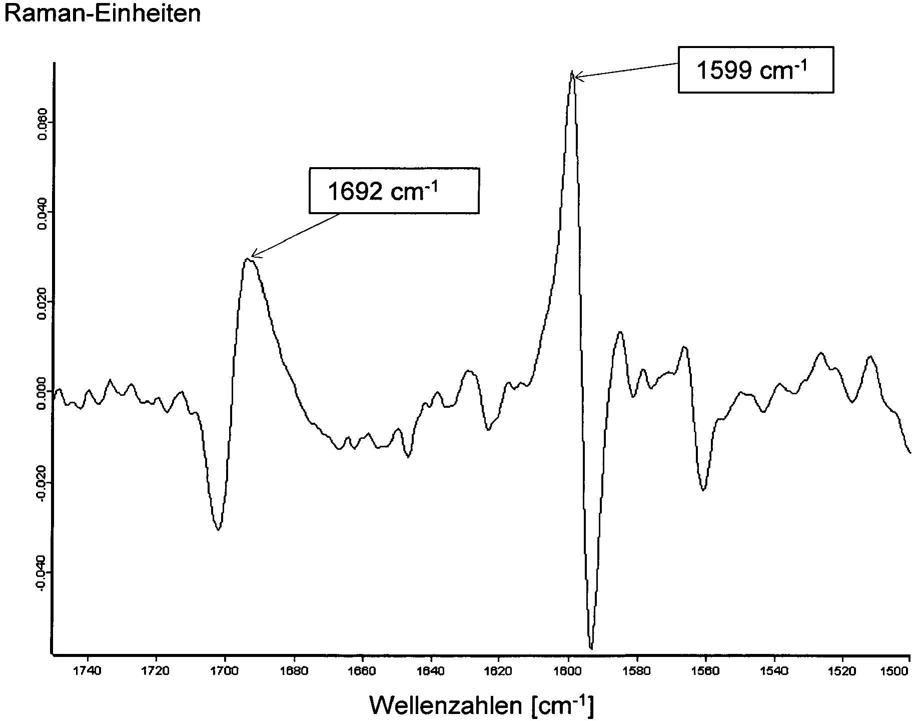 **Abbildung 1b** Differenz-Ramanspektrum (= Spektrum Tablette - **Spektrum** Vardenafil HCl 3 H₂O) von Tabletten des Beispieles 6 nach Rehydratisierung Vardenafil HCl wasserfrei rehydratisierte Tablette

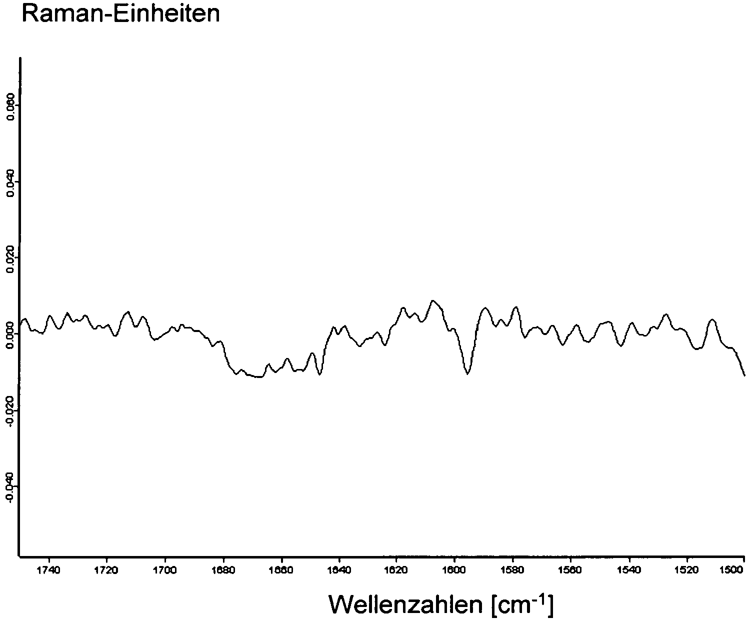

### Beispiel 7

37 kg Lacktabletten, die entsprechend Beispiel 6 hergestellt wurden, werden nach der Lackierung in einem Tablettenfass für 3 Tage über eine Begasungslanze mit 40 m³/h Zuluft von 21°C und 84 % relativer Feuchte behandelt.

### Beispiel 8

12,3 kg Vardenafil HCl Trihydrat, 30,2 kg mikrokristalline Cellulose und 2,24 kg gesiebtes Crospovidone werden gemischt. Diese Mischung wird mit weiteren 238 kg mikrokristalliner Cellulose und 12,8 kg gesiebtem Crospovidone gemischt, walz-granuliert und mit 1,5 kg hochdispersem Siliciumdioxid und 3,0 kg gesiebtem Magnesiumstearat nachgemischt. Diese Mischung wird auf einer Rundlauftablettenpresse zu runden Tabletten von 5,5 mm Durchmesser und einer Masse von 72 mg verpresst. Die Tabletten werden mit 5,74 kg Hypromellose, 1,91 kg Macrogol 400, 1,57 kg Titandioxid, 319 g Eisenoxid gelb und 25,5 g Eisenoxid rot lackiert. Die Lacktabletten werden in zwei Teilchargen zu etwa 140 kg rehydratisiert indem sie in einer Glatt Lackieranlage vom Typ 1250 für 5 Stunden mit 2000 m³/h Zuluft von 16 g Feuchte / kg bei 25°C (entsprechend 80 % relativer Luftfeuchte) behandelt werden.

### Beispiel 9

600 g Tabletten bestehend aus 5,926 mg Vardenafil HCl Trihydrat, 4,35 mg Crospovidone, 0,87 mg Magnesiumstearat, 75,419 mg mikrokristalliner Cellulose und 0,435 mg kolloidalem Siliciumdioxid werden in einem Kugelcoater mit einer organischen Lacklösung bestehend aus 6,65 % Celluloseacetat, 0,35 % PEG 3350, 92,535 % Aceton und 0,465 % Wasser beschichtet bis ein Lackauftrag von 82,76 g erreicht ist. Die lackierten Tabletten werden für 24 Stunden auf Hordenblechen bei 25°C / 80 % relativer Luftfeuchte behandelt.

### Beispiel 10

Es werden Tabletten wie im Beispiel 6 beschrieben hergestellt. Die Tabletten werden nach der Lackierung in Blisterpackungen eingesiegelt, welche aus 20 µm Aluminium- mit 3,5 g/m² PP- Verbundfolie und 300 µm farbloser und transparenter PP - Folie bestehen. Die Blisterpackungen werden über sechs Monate kontrolliert bei 25 °C und 60 % relativer Luftfeuchte inkubiert. Die Modifikation des Vardenafil HCl in den so behandelten Tabletten ***entspricht*** der Trihydratform.

### Beispiel 11

2,96 kg Vardenafil Hydrochlorid Trihydrat, 7,25 kg mikrokristalline Cellulose und 538 g Crospovidone werden in einem Pflugscharmischer gemischt. Die Mischung wird in einem Freifallmischer mit 26,1 kg mikrokristalliner Cellulose und 1,64 kg Crospovidone gemischt. Die Mischung wird auf einem Walzgranulator trocken granuliert und mit 4,35 kg mikrokristalliner Cellulose, 218 g hochdispersem Siliciumdioxid und 435 g Magnesiumstearat nachgemischt. Diese Mischung wird auf einer Tablettenpresse zu runden Tabletten von 6 mm Durchmesser verpresst. Die Tabletten werden in einer Lackiertrommel mit einer Lackierdispersion lackiert, welche folgende Bestandteile hat: 832 g Hypromellose, 277 g Macrogol 400, 227 g Titandioxid, 17,1 kg Wasser, 46,2 g gelbem und 3,70 g rotem Eisenoxid.

Die fertigen Tabletten werden in Blisterpackungen eingesiegelt, welche unter Verwendung einer 300 µm farblosen und transpartenten PP-Folie und 20 µm Aluminiumfolie mit Siegelschicht hergestellt wurden. In diesem Zustand entspricht die Modifikation von Vardenafil HCl in der Tablette nicht der Trihydratform. Die Blisterpackungen werden daher drei Tage lang bei 25 °C und 80 % Luftfeuchte inkubiert. Der Wirkstoff in der Tablette entspricht daraufhin dem Vardenafil Hydrochorid Trihydrat.

### Beispiel 12

Das Vorliegen der Trihydratform von Vardenafil HCl in der fertigen überzogenen Tablette wird durch FT Raman Spektroskopie untersucht. Das FT Raman Spektrum von Vardenafil HCl Trihydrat Tabletten zeichnet sich im Gegensatz zu entsprechenden Placebotabletten durch Banden bei 1701 cm⁻¹, 1624 cm⁻¹, 1594 cm⁻¹, 1580 cm⁻¹, 1561 cm⁻¹ und 1518 cm⁻¹ aus (Abb. 2). Die eindeutige Zugehörigkeit dieser Banden zum Wirkstoff Vardenafil HCl Trihydrat wird wie folgt belegt. Zu Vardenfil HCl Trihydrat existiert eine Einkristallröntgenstrukturanalyse mit deren Hilfe das theoretische zweidimensionale Röntgenpulverdiffraktogramm berechnet wird. Stimmt das von einer gegebenen Probe des Wirkstoffs experimentell erhaltene Röntgenpulverdiffraktogramm mit dem theoretischen Diffraktogramm überein handelt es sich bei der gegebenen Probe eindeutig um Vardenafil HCl Trihydrat. Das FT Raman Spektrum derselben Probe ist somit eindeutig Vardenafil HCl Trihydrat zuzuordnen (Tab.3). Wird diese Substanz getrocknet so treten Banden von 1 bis 4 Anhydratmodifikationen in FT Raman Spektrum auf, z.B. bei ca. 1692 cm⁻¹ und 1599 cm⁻¹. Entsprechend der Menge an in der Probe verblieben Vardenafil HCl Trihydrat reduziert sich die Intensität der zugehörigen Banden im FT Raman Spektrum (Abb.3). Die Abwesenheit der Banden bei ca. 1692 cm⁻¹ und 1599 cm⁻¹ im FT Raman Spektrum kann somit zur Prüfung auf vollständige Rehydratisierung von Vardenafil HCl Trihydrat herangezogen werden. Die praktische Vorgehensweise hierbei ist wie folgt: vom FT Raman Spektrum einer gegebenen Tablette wird das FT Raman Spektrum von Vardenfil HCl Trihydrat und das FT Raman Spektrum der wirkstofffreien Tablette der entsprechenden Formulierung subtrahiert. Verbleibende Raman Intensitäten, die grösser als das spektrale Rauschen und > 0 sind, sind Banden anderer Komponenten als der in der wirkstofffreien Tablette und Vardenafil HCl Trihydrat enthaltenen. In Fall von Vardenafil HCl Tabletten sind dies z.B. Banden von 1 bis 4 Anhydratmodifikation von Vardenafil HCl. **Abbildung 2:** Raman-Spektrum von Vardenafil HCl 3 H₂O

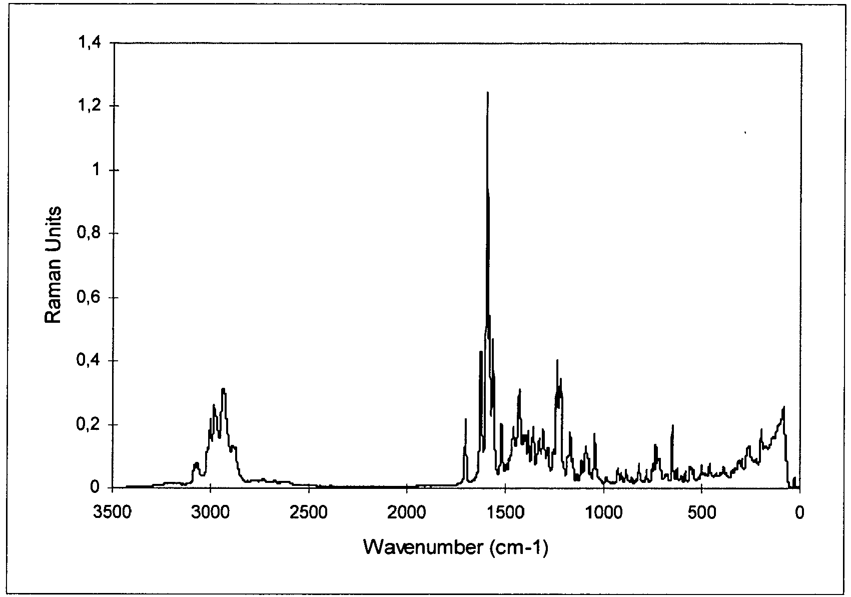

**Tabelle 3: Bandentabelle des Raman-Spektrums von Vardenafil HCl 3 H₂O**

| Peak [cm⁻¹] | | |
|---|---|---|
| 197 | 927 | 1424 |
| 260 | 1045 | 1456 |
| 297 | 1089 | 1517 |
| 313 | 1110 | 1561 |
| 390 | 1167 | 1581 |
| 460 | 1216 | 1594 |
| 499 | 1224 | 1623 |
| 583 | 1236 | 1701 |
| 624 | 1253 | 2940 |
| 649 | 1281 | 2983 |
| 733 | 1306 | 3002 |
| 746 | 1326 | |
| 778 | 1357 | |
| 817 | 1381 | |
| 882 | 1395 | |

**Abbildung 3:** Nachweis der Trihydratform von Vardenafil HCl in einer 20mg Tablette 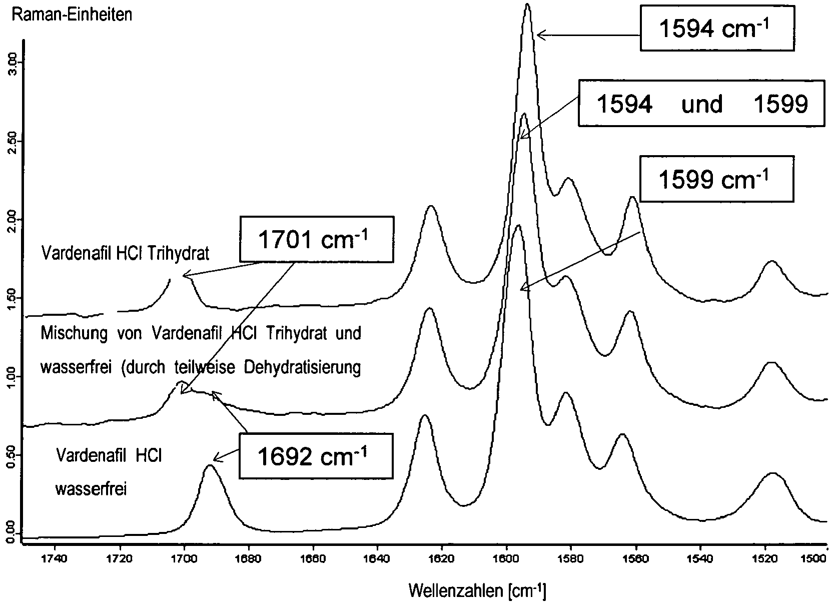 **Abbildung 4:** IR-Spektrum von Vardenafil HCl 3 H₂O

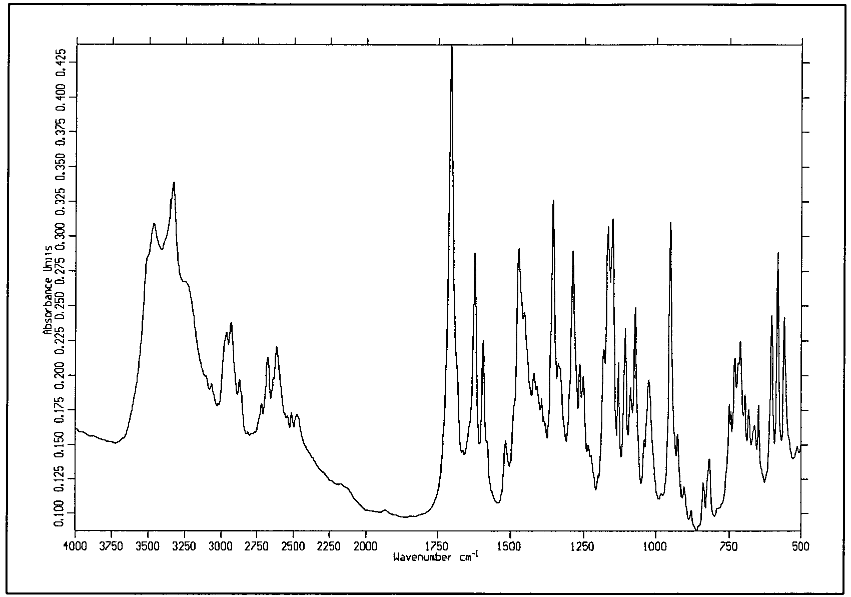

**Tabelle 4: Bandentabelle des IR-Spektrums von Vardenafil HCl 3 H₂O**

| Peak [cm⁻¹] | | | |
|---|---|---|---|
| 515 | 906 | 1253 | 1707 |
| 560 | 927 | 1264 | 1935 |
| 583 | 954 | 1289 | 2478 |
| 604 | 983 | 1339 | 2517 |
| 648 | 1026 | 1357 | 2544 |
| 663 | 1043 | 1382 | 2621 |
| 684 | 1074 | 1388 | 2641 |
| 697 | 1089 | 1395 | 2680 |
| 714 | 1108 | 1411 | 2722 |
| 721 | 1132 | 1421 | 2875 |
| 732 | 1153 | 1454 | 2934 |
| 745 | 1168 | 1475 | 2967 |
| 751 | 1182 | 1517 | 3068 |
| 820 | 1202 | 1580 | 3332 |
| 841 | 1226 | 1594 | 3467 |
| 882 | 1236 | 1623 | |

**Abbildung 5:** NIR-Spektrum von Vardenafil HCl 3 H₂O

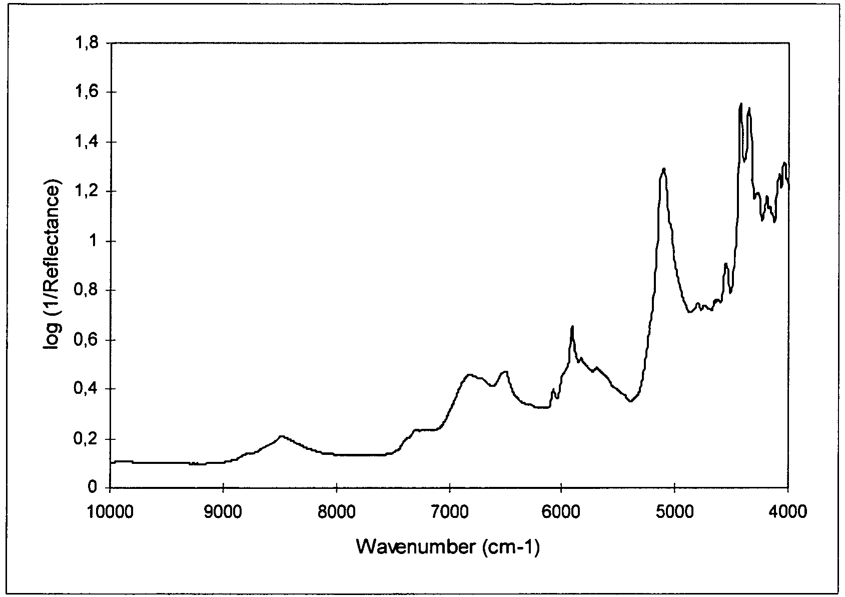

**Tabelle 5: Bandentabelle des NIR-Spektrums von Vardenafil HCl 3 H₂O**

| Peak [cm⁻¹] | |
|---|---|
| 4041 | 4804 |
| 4083 | 5107 |
| 4165 | 5694 |
| 4192 | 5833 |
| 4266 | 5911 |
| 4353 | 6080 |
| 4428 | 6501 |
| 4556 | 6818 |
| 4655 | 8479 |
| 4744 | |

**Abbildung 6:** FIR-Spektrum von Vardenafil HCl 3 H₂O

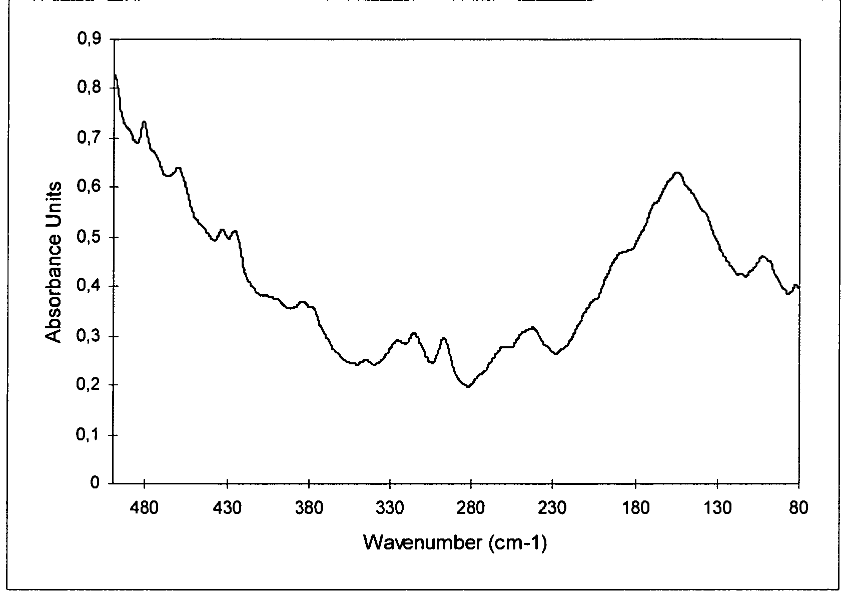

**Tabelle 6: Bandentabelle des FIR-Spektrums von Vardenafil HCl 3 H₂O**

| Peak [cm⁻¹] | |
|---|---|
| 82 | 326 |
| 102 | 345 |
| 115 | 384 |
| 154 | 426 |
| 243 | 434 |
| 259 | 460 |
| 297 | 481 |
| 315 | |

**Abbildung 7:** ¹³C-Festkörper NMR Spektrum von von Vardenafil HCl 3 H₂O

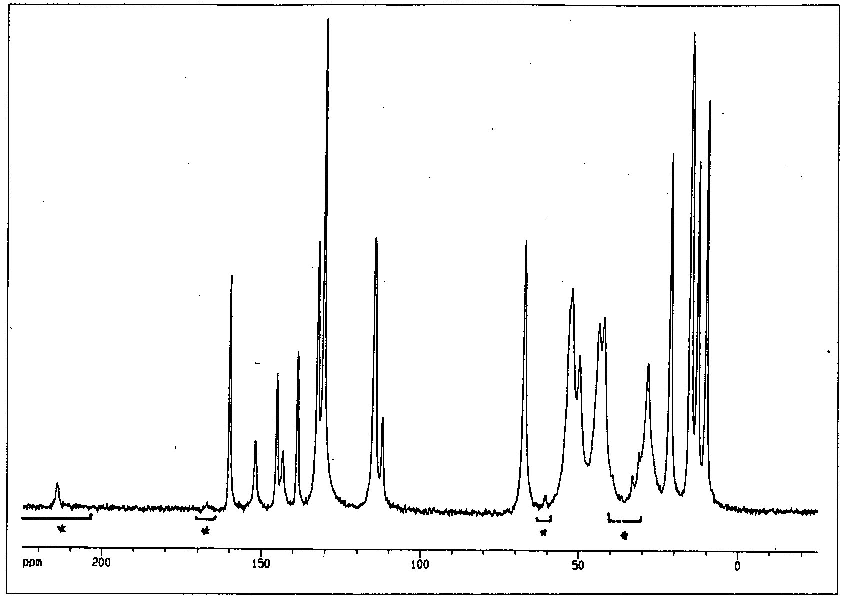

**Tabelle 7: Bandentabelle des ¹³C-Festkörper NMR Spektrums von Vardenafil HCl 3 H₂O**

| Peak [ppm] | |
|---|---|
| 10,6 | 67,7 |
| 13,3 | 112,3 |
| 15,4 | 115,0 |
| 15,7 | 130,8 |
| 21,7 | 132,6 |
| 28,7 | 138,8 |
| 31,3 | 143,3 |
| 42,6 | 145,3 |
| 44,1 | 152,1 |
| 50,2 | 160,2 |
| 52,8 | |

**Abbildung 8:** Röntgendiffraktogramm von Vardenafil HCl 3 H₂O

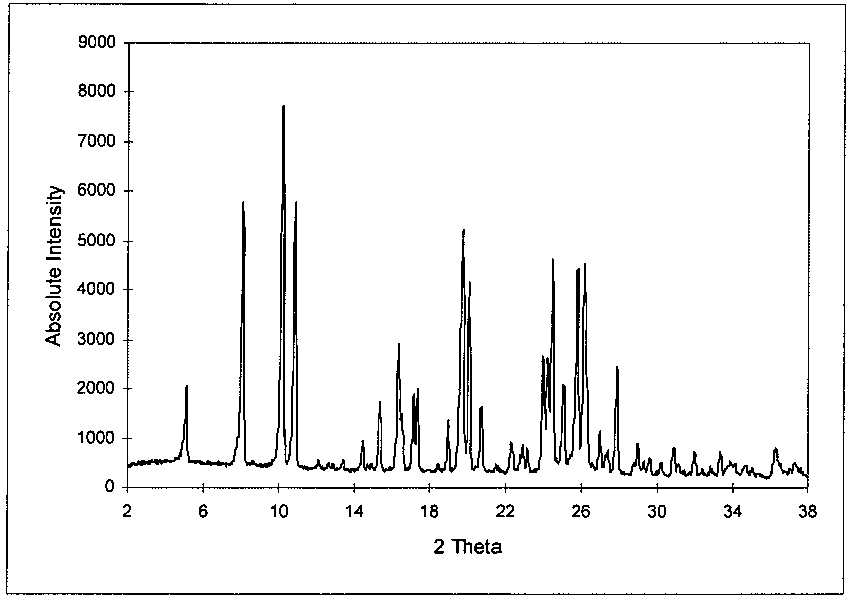

**Tabelle 8: Peakliste des Röntgendiffraktogramms von Vardenafil HCl 3 H₂O**

| Peak [2 Theta] | |
|---|---|
| 5,1 | 22,9 |
| 8,1 | 23,2 |
| 10,2 | 24,0 |
| 10,8 | 24,3 |
| 14,4 | 24,5 |
| 15,3 | 25,1 |
| 16,3 | 25,8 |
| 17,2 | 26,2 |
| 17,4 | 27,0 |
| 19,0 | 27,9 |
| 19,7 | 29,0 |
| 20,1 | 30,9 |
| 20,7 | 32,0 |
| 22,3 | 33,3 |

## Patentansprüche

1. Verfahren zur Herstellung von überzogenen Tabletten enthaltend Vardenafil Hydrochlorid mit einem Trihydrat-Gehalt von mindestens 90 Mol-% in fester Form, **dadurch gekennzeichnet, dass**
a) bei der Herstellung der überzogenen Tabletten Vardenafil Hydrochlorid mit beliebigem Wassergehalt eingesetzt wird, und
b) im Endprodukt das Vardenafil Hydrochlorid in die Trihydratform überführt wird, indem das Endprodukt so lange mit befeuchtetem Gas in Kontakt gebracht wird, bis sich zu mindestens 90 Mol-% das Trihydrat gebildet hat.

2. Verfahren gemäß Anspruch 1, **dadurch gekennzeichnet, dass** die Überführung in die Trihydratform mit befeuchtetem Gas in einer Zeitspanne von wenigen Minuten bis zu mehreren Tagen erfolgt.

3. Verfahren gemäß Anspruch 1, **dadurch gekennzeichnet, dass** die Überführung in die Trihydratform mit befeuchtetem Gas in einer Zeitspanne von 0,5 Stunden bis 12 Stunden erfolgt

4. Verfahren gemäß Anspruch 1, **dadurch gekennzeichnet, dass** die Überführung in die Trihydratform in pharmazeutisch üblichen Blisterpackungen mit befeuchtetem Gas in einer Zeitspanne von 1 Tag bis zu 6 Monaten erfolgt.

5. Verfahren gemäß mindestens einem der Ansprüche 1 bis 4, **dadurch gekennzeichnet, dass** die Überführung in die Trihydratform mit Luft mit einer relativen Feuchte von 35% bis 100% erfolgt.

6. Verfahren gemäß mindestens einem der Ansprüche 1 bis 4, **dadurch gekennzeichnet, dass** die Überführung in die Trihydratform mit Luft mit einer relativen Feuchte von 50% bis 99% erfolgt.

7. Überzogene Tabletten erhältlich nach dem Verfahren gemäß mindestens einem der Ansprüche 1 bis 6, enthaltend Vardenafil Hydrochlorid Trihydrat zu 0,1-70 Gewichts-%, ein Sprengmittel zu 0,1-10 Gewichts-%, ein Schmiermittel zu 0,1-2 Gewichts-% und gegebenenfalls weitere Hilfsmittel sowie ein Füllmittel als restliche Bestandteile.

8. Überzogene Tabletten gemäß Anspruch 7, enthaltend mikrokristalline Cellulose als Füllmittel, als Sprengmittel Crospovidone und als Schmiermittel Magnesiumstearat.

9. Überzogene Tabletten nach Anspruch 7 oder 8 zur Behandlung der erektilen Dysfunktion.

## Claims

1. Method for producing coated tablets comprising vardenafil hydrochloride at not less than 90 mol% as trihydrate in solid form, **characterized in that**
a) vardenafil hydrochloride with any content of water is employed in the production of the coated tablets, and
b) the vardenafil hydrochloride is converted into the trihydrate form in the final product by bringing the final product into contact with moistened gas until the trihydrate has formed to an extent of not less than 90 mol%.

2. Method according to Claim 1, **characterized in that** the conversion into the trihydrate form with moistened gas is effected in the course of a time interval ranging from a few minutes up to several days.

3. Method according to Claim 1, **characterized in that** the conversion into the trihydrate form with moistened gas is effected in the course of a time interval ranging from 0.5 hours to 12 hours.

4. Method according to Claim 1, **characterized in that** the conversion into the trihydrate form with moistened gas is effected in pharmaceutically customary blister packs in the course of a time interval ranging from 1 day up to 6 months.

5. Method according to at least one of Claims 1 to 4, **characterized in that** the conversion into the trihydrate form is effected with air having a relative humidity of from 35% to 100%.

6. Method according to at least one of Claims 1 to 4, **characterized in that** the conversion into the trihydrate form is effected with air having a relative humidity of from 50% to 99%.

7. Coated tablets obtainable by the method according to at least one of Claims 1 to 6, comprising vardenafil hydrochloride trihydrate at 0.1-70% by weight, a disintegrant at 0.1-10% by weight, a lubricant at 0.1-2% by weight and optionally further adjuncts and also a filler as remaining ingredients.

8. Coated tablets according to Claim 7, comprising microcrystalline cellulose as filler, crospovidone as disintegrant and magnesium stearate as lubricant.

9. Coated tablets according to Claim 7 or 8 for the treatment of erectile dysfunction.

## Revendications

1. Procédé de fabrication de comprimés enrobés contenant du chlorhydrate de vardénafil ayant une teneur en trihydrate d'au moins 90 % en moles sous forme solide, **caractérisé en ce que**
a) lors de la fabrication des comprimés enrobés, du chlorhydrate de vardénafil ayant une teneur en eau quelconque est utilisé, et
b) dans le produit final, le chlorhydrate de vardénafil est transformé en la forme trihydratée, par mise en contact du produit final avec un gaz humidifié jusqu'à ce qu'au moins 90 % en moles du trihydrate se soit formé.

2. Procédé selon la revendication 1, **caractérisé en ce que** la transformation en la forme trihydratée a lieu avec un gaz humidifié en une période de temps allant de quelques minutes à plusieurs jours.

3. Procédé selon la revendication 1, **caractérisé en ce que** la transformation en la forme trihydratée a lieu avec un gaz humidifié en une période de temps allant de 0,5 heure à 12 heures.

4. Procédé selon la revendication 1, **caractérisé en ce que** la transformation en la forme trihydratée a lieu dans des plaquettes thermoformées pharmaceutiquement usuelles avec un gaz humidifié en une période de temps allant de 1 jour à 6 mois.

5. Procédé selon au moins l'une quelconque des revendications 1 à 4, **caractérisé en ce que** la transformation en la forme trihydratée a lieu avec de l'air ayant une humidité relative de 35 % à 100 %.

6. Procédé selon au moins l'une quelconque des revendications 1 à 4, **caractérisé en ce que** la transformation en la forme trihydratée a lieu avec de l'air ayant une humidité relative de 50 % à 99 %.

7. Comprimés enrobés pouvant être obtenus par le procédé selon au moins l'une quelconque des revendications 1 à 6, contenant du chlorhydrate de vardénafil trihydraté à hauteur de 0,1 à 70 % en poids, un désintégrant à hauteur de 0,1 à 10 % en poids, un lubrifiant à hauteur de 0,1 à 2 % en poids et éventuellement d'autres adjuvants, ainsi qu'une charge en tant que constituants restants.

8. Comprimés enrobés selon la revendication 7, contenant de la cellulose microcristalline en tant que charge, de la crospovidone en tant que désintégrant et du stéarate de magnésium en tant que lubrifiant.

9. Comprimés enrobés selon la revendication 7 ou 8 pour le traitement du dysfonctionnement érectile.
